(19) Europäisches Patentamt
European Patent Office
Office européen des brevets

(11) Veröffentlichungsnummer: **0 007 987 A1**

(12) **EUROPÄISCHE PATENTANMELDUNG**

(21) Anmeldenummer: **79101944.1**

(22) Anmeldetag: **15.06.79**

(51) Int. Cl.[3]: **C 07 C 149/14**, A 01 N 47/24, C 07 D 295/22

(30) Priorität: **27.06.78 DE 2828133**

(43) Veröffentlichungstag der Anmeldung: **20.02.80 Patentblatt 80/4**

(84) Benannte Vertragsstaaten: **AT BE CH DE FR GB IT NL SE**

(71) Anmelder: **BAYER Aktiengesellschaft, Zentralbereich Patente,Marken und Lizenzen Bayerwerk, D-5090 Leverkusen 1 (DE)**

(72) Erfinder: **Stetter, Jörg, Dr., Pahlkestrasse 3, D-5600 Wuppertal-1 (DE)**
Erfinder: **Homeyer, Bernard, Dr., Obere Strasse 28, D-5090 Leverkusen (DE)**
Erfinder: **Hammann, Ingeborg, Dr., Belfortstrasse 9, D-5000 Köln 1 (DE)**

(54) **N-sulfenylierte Carbamoyloximino-1-methylthio-butane, Verfahren zu ihrer Herstellung und ihre Verwendung als Insektizide.**

(57) Die vorliegende Erfindung betrifft neue N-sulfenylierte Carbamoyloximino-1-methylthio-butane der allgemeinen Formel (I)

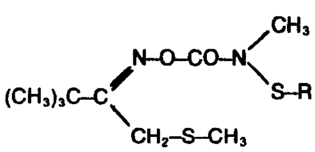
(I)

in welcher R die in der Beschreibung angegebene Bedeutung besitzt.

Sie weisen starke insektizide, insbesondere auch wurzelsystemische Eigenschaften auf.

Man erhält sie, wenn man

a) das Oxim der Formel II

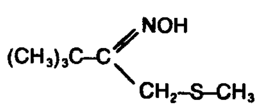
(II)

mit sulfenylierten Carbamoylhalogeniden der Formel

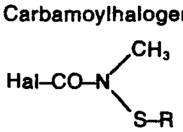
(III)

umsetzt, oder

b) gegebenenfalls die nach dem Verfahren (a) erhältlichen sulfenylierten Oximcarbamate der Formel

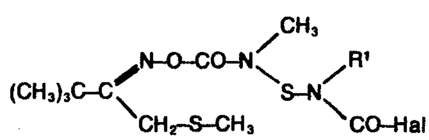
(IV)

mit Verbindungen der Formel

$H{-}R^8$

umsetzt.

EP 0 007 987 A1

BAYER AKTIENGESELLSCHAFT
Zentralbereich
Patente, Marken und Lizenzen

5090 Leverkusen, Bayerwerk
Rt/Kü
Ib

N-sulfenylierte Carbamoyloximino-1-methylthio-butane, Verfahren zu ihrer Herstellung und ihre Verwendung als Insektizide

Die vorliegende Erfindung betrifft neue N-sulfenylierte Carbamoyloximino-1-methylthio-butane, mehrere Verfahren zu ihrer Herstellung und ihre Verwendung als Insektizide.

Es ist bereits bekannt geworden, daß Oximcarbamate, wie beispielsweise 1-Cyan-2-methylpropanaldoxim-N-methylcarbamat und 1-Cyan-butanaldoxim-N-methylcarbamat oder 3,3-Dimethyl-2-methylcarbamoyloximino-1-methylthio-butan (Dacamox) pestizide, insbesondere insektizide und nematozide Eigenschaften besitzen (vergleiche Deutsche Offenlegungsschriften 1 567 142 bzw. 2 216 838). Deren Wirkung ist jedoch, insbesondere bei niedrigen Aufwandmengen, nicht immer ganz befriedigend.

Es wurden neue N-sulfenylierte Carbamoyloximino-1-methylthio-butane der allgemeinen Formel (I)

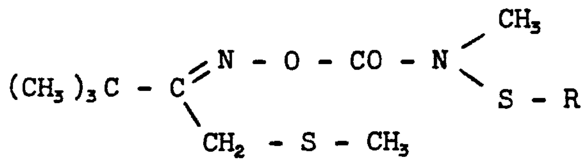

(I)

in welcher

R für Alkyl, Halogenalkyl, gegebenenfalls substituiertes Phenyl oder die Gruppe $-NR^1R^2$ sowie für den gleichen Rest steht, an den die Gruppe -SR gebunden ist,

$R^1$ für Alkyl steht,

$R^2$ für Alkyl, Halogencarbonyl, gegebenenfalls substituiertes Phenylsulfonyl oder die Gruppierungen $-CO-O-R^3$ und $-CO-NR^4R^5$ steht sowie

$R^1$ und $R^2$ gemeinsam mit dem N-Atom für einen Ring stehen, der gegebenenfalls ein weiteres Heteroatom enthält,

$R^3$ für Alkyl, Alkenyl, Alkinyl, gegebenenfalls substituiertes Aryl oder die Gruppierung $-N=CR^6R^7$ steht,

$R^4$ und $R^5$ gleich oder verschieden sind und für Alkyl stehen oder gemeinsam mit dem N-Atom für einen Ring stehen, der gegebenenfalls ein weiteres Heteroatom enthält,

$R^6$ und $R^7$ gleich oder verschieden sind und für Wasserstoff, Alkyl, Alkoxy, Alkylthio, Alkoxyalkyl, Alkylthioalkyl, Dialkylaminocarbonyl und Alkoxycarbonyl oder gemeinsam mit dem C-Atom für einen Dithian-Ring stehen,

gefunden. Sie weisen starke insektizide, insbesondere auch wurzelsystemische Eigenschaften auf.

Die Verbindungen der Formel (I) können in der syn- oder anti-Form vorliegen; vorwiegend fallen sie als Gemische beider Formen an.

Man erhält die N-sulfenylierten Carbamoyloximino-1-methylthio-butane der Formel (I), wenn man

a) das Oxim der Formel (II)

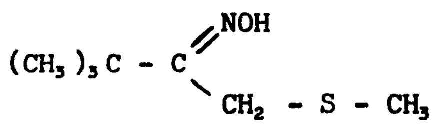

(II)

mit sulfenylierten Carbamoylhalogeniden der Formel (III)

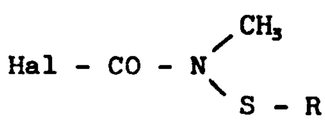

(III)

in welcher

R die oben angegebene Bedeutung hat und

Hal für Fluor oder Chlor steht,

in Gegenwart eines Verdünnungsmittels und eines Säurebindemittels umsetzt, oder

b) gegebenenfalls die nach dem Verfahren (a) erhältlichen sulfenylierten Oximcarbamate der Formel (IV)

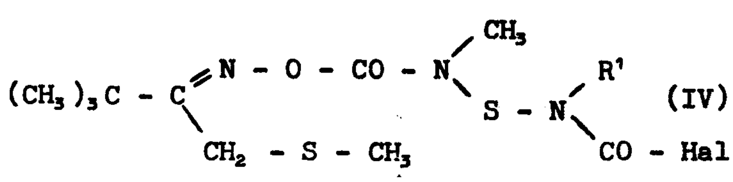

(IV)

in welcher

$R^1$ und Hal die oben angegebene Bedeutung haben,

mit Verbindungen der Formel (V)

$$H - R^8 \qquad (V)$$

in welcher

$R^8$ für die Gruppierungen $-OR^3$ und $-NR^4R^5$ steht und $R^3$, $R^4$ und $R^5$ die oben angegebene Beutung haben,

in Gegenwart eines Verdünnungsmittels und eines Säurebindemittels umsetzt.

Ueberraschenderweise zeigen die erfindungsgemäßen N-sulfenylierten Carbamoyloximino-1-methylthio-butane eine höhere insektizide und wurzelsystemische Wirkung als die bekannten Oximcarbamate 1-Cyan-2-methylpropanaldoxim-N-methylcarbamat, 1-Cyan-butanaldoxim-N-methylcarbamat und 3,3-Dimethyl-2-methyl-carbamoyloximino-1-methylthio-butan, welches chemisch und wirkungsmäßig naheliegende Verbindungen sind. Die erfindungsgemäßen Wirkstoffe stellen somit eine Bereicherung der Technik dar.

Die erfindungsgemäßen N-sulfenylierten Carbamoyloximino-1-methylthio-butane sind durch die Formel (I) allgemein definiert. In dieser Formel steht R vorzugsweise für Alkyl mit 1 bis 4 Kohlenstoffatomen, Halogenalkyl mit 1 bis 2 Kohlenstoff- und bis zu 5 Halogenatomen, wie insbesondere Fluor- und Chloratomen, gegebenenfalls substituiertes Phenyl, wobei als Substituenten vorzugsweise genannt seien: Halogen, insbesondere Fluor, Chlor oder Brom, Nitro, Cyano, Alkyl mit 1 bis 2 Kohlenstoffatomen und Halogenalkyl mit 1 bis 2 Kohlenstoff- und bis zu 5 Halogenatomen, wie insbesondere Fluor- und Chloratomen. R steht außerdem vorzugsweise für die Gruppe $-NR^1R^2$ sowie für den gleichen Rest, an den die Gruppe -SR gebunden ist. $R^1$ steht vorzugsweise für geradkettiges oder verzweigtes Alkyl mit 1 bis 4 Kohlenstoffatomen. $R^2$ steht vorzugsweise für geradkettiges oder verzweigtes Alkyl mit 1 bis 4 Kohlenstoffatomen, Halogencarbonyl mit Fluor und Chlor als Halogenatomen, sowie gegebenenfalls substituiertes Phenylsulfonyl, wobei als Subsstituenten vorzugsweise infrage kommen: ·Halogen, insbesondere Fluor, Chlor oder Brom, Alkyl mit 1 bis 2 Kohlenstoffatomen, Halogenalkyl mit 1 bis 2 Kohlenstoff- und bis zu 5 Halogenatomen, wie insbesondere Fluor- oder Chloratomen, Cyano oder Nitro. $R^2$ steht außerdem vorzugsweise für die Gruppierungen -CO-O-$R^3$ und -CO-N$R^4R^5$. $R^1$ und $R^2$ stehen außerdem zusammen mit dem N-Atom noch vorzugsweise für einen 5- bis 7-gliedrigen Ring, der gegebenenfalls ein weiteres Heteroatom enthalten kann, wie insbesondere Sauerstoff oder Stickstoff. $R^3$ steht vorzugsweise für Alkyl mit 1 bis 4 Kohlenstoffatomen, Alkenyl und Alkinyl mit jeweils 3 bis 4 Kohlenstoffatomen, sowie gegebenenfalls einfach oder mehrfach, gleich oder verschieden substituiertes Aryl mit 6 bis 10 Kohlenstoffatomen, wobei als Substituenten vorzugsweise infrage kommen: Halogen, geradkettiges oder verzweigtes Alkyl mit 1 bis 5 Kohlenstoffatomen, Alkoxy und Alkylthio mit jeweils 1 bis 4 Kohlenstoffatomen, Dialkylamino mit 1 bis 4 Kohlenstoffatomen in jedem Alkylteil, Dialkylamino mit 2 bis 4 Kohlenstoffatomen in jedem Alkenylteil, Alkylthioalkyl mit 1 bis 2 Kohlenstoffatomen in jedem Alkylteil sowie gegebenenfalls ein- oder mehrfach durch Alkyl mit 1 bis 2 Kohlenstoffatomen substituiertes Dioxolanyl. $R^3$ steht außerdem vorzugsweise für gegebenenfalls ein- oder mehrfach durch Alkyl mit 1 bis 2 Kohlenstoffatomen substituiertes Benzofuranyl und Benzodioxolanyl. $R^3$ steht weiterhin vorzugsweise für die Gruppierung -N-C$R^6R^7$.
$R^4$ und $R^5$ sind gleich oder verschieden und stehen vorzugsweise für Alkyl mit 1 oder 2 Kohlenstoffatomen sowie gemeinsam mit dem N-Atom für einen 5- bis 7-gliedrigen Ring, der gegebenenfalls ein weiteres Heteroatom enthalten kann. $R^6$ und $R^7$ sind gleich oder verschieden und stehen vorzugsweise für Wasserstoff, geradkettiges oder verzweigtes Alkyl mit 1 bis 4 Kohlenstoffatomen; Alkylthio, Alkoxy,

Alkylthioalkyl und Alkoxyalkyl mit jeweils 1 bis 4 Kohlenstoffatomen in jedem Alkylteil, Dialkylaminocarbonyl mit 1 bis 2 Kohlenstoffatomen in jedem Alkylteil sowie Alkoxycarbonyl mit 1 bis 4 Kohlenstoffatomen im Alkylteil. $R^6$ und $R^7$ stehen außerdem zusammen mit dem C-Atom noch vorzugsweise für einen Dithian-Ring.

Ganz besonders bevorzugt sind diejenigen N-sulfenylierten Carbamoyloximino-1-methylthio-butane der Formel (I), in denen R für Trifluormethyl, Chlor-difluor-methyl, Dichlorfluor-methyl, Trichlormethyl, 4-Chlorphenyl, 3-Trifluormethylphenyl sowie die Gruppe $-NR^1R^2$ und den gleichen Rest steht, an den die Gruppe -SR gebunden ist; $R^1$ für Methyl steht; $R^2$ für Methyl, Fluorcarbonyl, 4-Methylphenylsulfonyl sowie die Gruppierungen $-CO-O-R^3$ und $-CO-NR^4R^5$ steht; $R^1$ und $R^2$ zusammen mit dem N-Atom für Piperidino oder Morpholino stehen; $R^3$ für Methyl, Ethyl, Allyl, Propargyl, 2,2-Dimethylbenzofuran-7-yl, 2,2-Dimethyl-1,3-benzodioxol-4-yl, Naphth-1-yl und gegebenenfalls einfach oder mehrfach gleich oder verschieden substituiertes Phenyl steht, wobei als Substituenten besonders bevorzugt infrage kommen: Chlor, Methyl, Ethyl, i-Propyl, i-Butyl, sek.-Butyl, tert.-Butyl, Pent-2-yl, Pent-3-yl, Methoxy, Methylmercapto, i-Propoxy, i-Propylmercapto, Methoxymethyl, Ethoxymethyl, Methylthiomethyl, Ethylthiomethyl, Dimethylamino, Diallylamino und 4,5-Dimethyl-1,3-dioxolan-2-yl; $R^3$ weiterhin für die Gruppierung $-N=CR^6R^7$ steht; $R^4$ und $R^5$ für Methyl oder zusammen mit dem N-Atom für Piperidino und Morpholino stehen; $R^6$ und $R^7$ gleich oder verschieden sind und für Wasserstoff, Methyl, i-Propyl, tert.-Butyl, Methoxy, Methylmercapto, Methoxymethyl, Methylthiomethyl, Dimethylcarbamoyl und Methoxycarbonyl stehen, oder gemeinsam mit dem C-Atom für einen 1,3-Dithian-Ring stehen.

Verwendet man 3,3-Dimethyl-1-methylthio-2-oximino-butan und N-Methyl-N-morpholin-4-yl-sulfenyl-carbamoylfluorid als Ausgangsstoffe, so kann der Reaktionsablauf durch das folgende Formelschema wiedergegeben werden (Verfahren a):

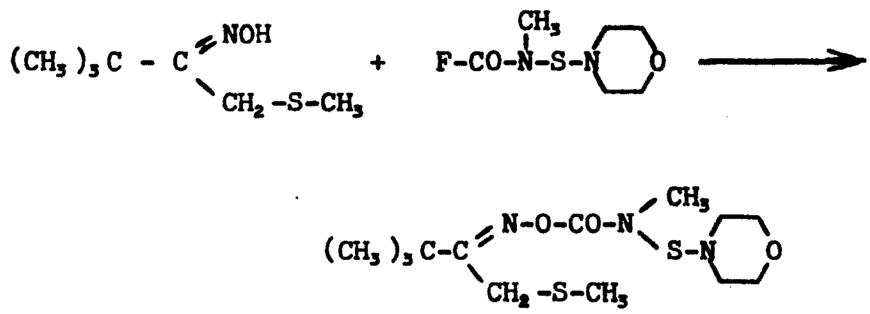

Verwendet man 2 Mol 3,3-Dimethyl-1-methylthio-2-oximino-butan und 1 Mol N,N'-Bis-(fluorcarbonyl)-thio-bis-methylamin als Ausgangsstoffe, so kann der Reaktionsablauf durch das folgende Formelschema wiedergegeben werden (Verfahren a):

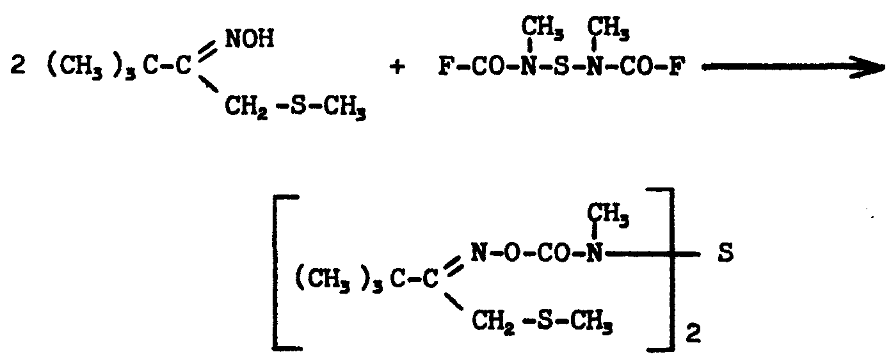

Verwendet man 1 Mol 3,3-Dimethyl-1-methylthio-2-oximino-butan und 1 Mol N,N'-Bis-(fluorcarbonyl)-thio-bis-methylamin als Ausgangsstoffe, so kann der Reaktionsablauf durch das folgende Formelschema wiedergegeben werden (Verfahren a):

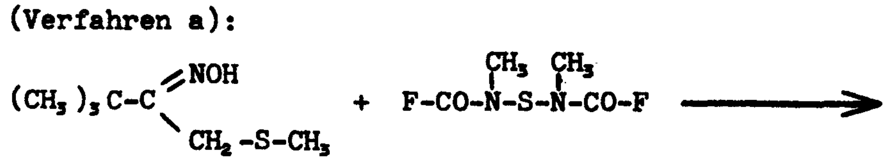

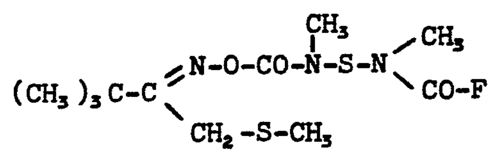

Verwendet man 3,3-Dimethyl-1-methylthio-2-[N-methyl-N-(N'-methyl-N'-fluorcarbonyl)-aminosulfonyl]-carbamoyloximino-butan und Methanol als Ausgangsstoffe, so kann der Reaktionsablauf durch das folgende Formelschema wiedergegeben werden (Verfahren b):

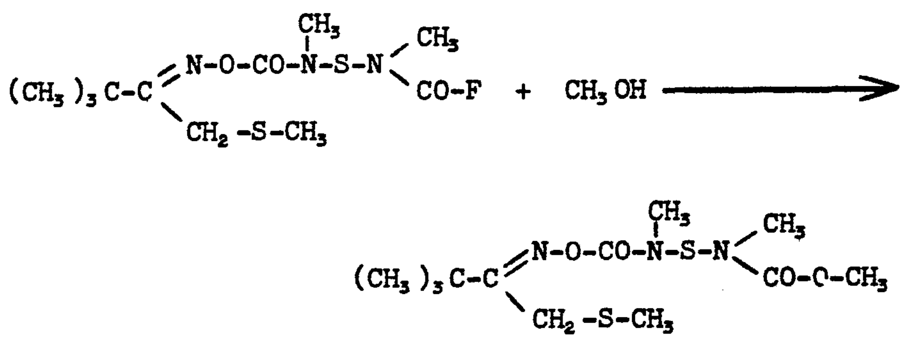

Das für die Verfahrensvariante (a) als Ausgangsstoff zu verwendende Oxim der Formel (II) ist bekannt (vergleiche Deutsche Offenlegungsschrift 2 216 838).

Die außerdem für die Verfahrensvariante (a) als Ausgangsstoffe benötigten sulfenylierten Carbamoylhalogenide sind durch die Formel (III) allgemein definiert. In dieser Formel steht R vorzugsweise für die Reste, die bei den Verbindungen der Formel (I) bereits vorzugsweise genannt wurden.

Die sulfenylierten Carbamoylchloride der Formel (III) sind bekannt (vergleiche Deutsche Offenlegungsschriften 2 517 653 und 2 654 282) oder lassen sich nach allgemein üblichen und bekannten Verfahren herstellen. Man erhält sie z.B. durch Umsetzung der entsprechenden Carbamidsäurehalogenide der Formel (VI)

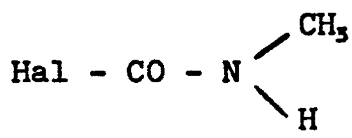

(VI)

in welcher

Hal die oben angegebene Bedeutung hat,

mit den entsprechenden Sulfenchloriden der Formel (VII)

$Cl - S - R$ (VII)

in welcher

R die oben angegebene Bedeutung hat,

in Gegenwart eines säurebindenden Mittels (vergleiche hierzu auch die Angaben in der Deutschen Auslegeschrift 1 297 095, den Deutschen Offenlegungsschriften 2 357 930, 2 409 463 und 2 654 282 sowie der US-Patentschrift 3 939 192).

Als Ausgangsstoffe der Formel (III) seien beispielsweise genannt:

N,N'-Bis-(fluorcarbonyl)-thio-bis-methylamin
N-Methyl-N-morpholinylsulfenyl-carbamoylfluorid
1-Methylthioacetaldehyd-O-[N-methyl-N-(N'-methyl-N'-fluorformyl-aminosulfenyl)-carbamoyl]-oxim
N-Methyl-N-(N'-methyl-N'-methoxycarbonyl-aminosulfenyl)-carbamoylfluorid
N-Methyl-N-(N'-methyl-N'-äthoxycarbonyl-aminosulfenyl)-carbamoylfluorid
N-Methyl-N-[N'-methyl-N'-(2-isopropoxy-phenoxycarbonyl)-aminosulfenyl]-carbamoylfluorid
N-Methyl-N-[N'-methyl-N'-(3,5-dimethyl-4-dimethylamino-phenoxycarbonyl)-aminosulfenyl]-carbamoylfluorid
N-Methyl-N-[N'-methyl-N'-3,5-dimethyl-4-methylmercapto-phenoxycarbonyl)-aminosulfenyl]-carbamoylfluorid
N-Methyl-N-[N'-methyl-N'-(2-sek.-butyl-phenoxycarbonyl)-aminosulfenyl]-carbamoylfluorid
N-Methyl-N-[N'-methyl-N'-(3-methyl-4-dimethylamino-phenoxycarbonyl)-aminosulfenyl]-carbamoylfluorid
N-Methyl-N-trichlormethylsulfenyl-carbamoylfluorid
N-Methyl-N-fluordichlormethylsulfenyl-carbamoylfluorid
N-Methyl-N-chlordifluormethylsulfenyl-carbamoylfluorid
N-Methyl-N-(4-chlorphenylsulfenyl)-carbamoylfluorid
N-Methyl-N-(3-trifluormethylphenylsulfenyl-carbamoyl-fluorid
N-Methyl-N-[(4-methylphenylsulfonyl)-methyl-amino-sulfenyl]-carbamoylfluorid

Die für die erfindungsgemäße Umsetzung (b) als Ausgangsstoffe zu verwendenden Verbindungen sind durch die Formel (V) allgemein definiert. In dieser Formel steht $\underline{R^8}$ vorzugsweise für die Gruppierungen $-O-R^3$ und $-NR^4R^5$, wobei $\underline{R^3}$, $\underline{R^4}$ und $\underline{R^5}$ vorzugsweise für die Reste stehen, die bei den Verbindungen der Formel (I) bereits vorzugsweise genannt wurden.

Die Verbindungen der Formel (V) sind allgemein bekannte Verbindungen der organischen Chemie. Als Beispiele seien genannt:
Methanol, Aethanol, Isopropanol, Allylalkohol, Propargylalkohol, Phenol; 2,2-Dimethyl-benzofuran-7-ol; 2,2-Dimethyl-1,3-benzodioxo-4-ol, Naphthol; 3,5-Dimethyl-4-dimethylamino-phenol; 3,5-Dimethyl-4-methylmercaptophenol; 2-sek.-Butyl-phenol; 3-Methyl-4-dimethylaminophenol; 2-Isopropoxy-phenol; 2-Chlor-4,5-dimethyl-phenol; 3-Isopropyl-5-methyl-phenol; 3,5-Di-tert.-butyl-phenol; 3,5-Dimethyl-4-bis-allyl-amino-phenol; 3,4-Dimethylphenol; 3-Methylphenol; 3,4,5-Trimethylphenol; 2-Chlorphenol; 2-Isopropylphenol; 3-tert.-Butylphenol; 2-Aethylthiomethylphenol.

Als Verdünnungsmittel kommen für das erfindungsgemäße Verfahren (a) vorzugsweise alle inerten organischen Lösungsmittel infrage. Hierzu gehören vorzugsweise Ketone, wie Diethylketon, insbesondere Aceton und Methylethylketon; Nitrile, wie Propionitril, insbesondere Acetonitril; Alkohole, wie Ethanol oder Isopropanol; Ether, wie Tetrahydrofuran oder Dioxan; Formamide, wie insbesondere Dimethylformamid und halogenierte Kohlenwasserstoffe, wie Methylenchlorid, Tetrachlorkohlenstoff oder Chloroform.

Die Umsetzungen gemäß Verfahren (a) und (b) werden in Gegenwart eines Säurebinders vorgenommen. Man kann alle üblicherweise verwendbaren anorganischen und organischen Säurebinder zugeben. Hierzu gehören vorzugsweise Alkalicarbonate, wie beispielsweise Natriumcarbonat, Kaliumcarbonat und Natriumhydrogencarbonat, ferner niedere tertiäre Alkylamine, Cycloalkylamine oder Arylalkylamine, wie beispielsweise Triethylamin, N,N-Dimethyl-benzylamin, Dicyclohexylamin, weiterhin Pyridin und Diazabicyclooctan.

Die Reaktionstemperaturen können bei der Durchführung des Verfahrens (a) in einem größeren Bereich variiert werden. Im allgemeinen arbeitet man zwischen 0 und 100°C, vorzugsweise zwischen 10 und 80°C.

Bei der Durchführung des Verfahrens (a) setzt man vorzugsweise auf 1 Mol des Oxims der Formel (II) 1 bis 2 Mol, für den Fall eines dimeren Produktes 0,5 Mol, an Carbamoylhalogenid der Formel (III) und 1 bis 2 Mol Säurebinder ein. Die Isolierung der Verbindungen der Formel (I) erfolgt in üblicher und bekannter Weise.

Für das erfindungsgemäße Verfahren (b) kommen als Verdünnungsmittel vorzugsweise inerte organische Lösungsmittel infrage. Hierzu gehören vorzugsweise Ether, wie Diethylether und Dioxan; aromatische Kohlenwasserstoffe, wie Benzol und Toluol; in einzelnen Fällen auch chlorierte Kohlenwasserstoffe, wie Chloroform, Methylenchlorid oder Tetrachlorkohlenstoff; ferner Ester, wie Essigsäureethylester, sowie Hexamethylphosphorsäuretriamid. In manchen Fällen kann auch ein Ueberschuß der Verbindung der Formel (V) verwendet werden.

Die Reaktionstemperaturen können bei der Durchführung des Verfahrens (b) in einem größeren Bereich variiert werden. Im allgemeinen arbeitet man zwischen 0 und 100°C, vorzugsweise zwischen 10 und 60°C.

Bei der Durchführung des Verfahrens (b) werden die Ausgangsstoffe vorzugsweise in molaren Mengen eingesetzt. Die Isolierung der Verbindungen der Formel (I) erfolgt nach üblichen Methoden.

Die Wirkstoffe eignen sich bei guter Pflanzenverträglichkeit und günstiger Warmblütertoxizität zur Bekämpfung von tierischen Schädlingen, insbesondere Insekten, Spinnentieren und Nematoden, die in der Landwirtschaft, in Forsten, im Vorrats- und Materialschutz sowie auf dem Hygienesektor vorkommen. Sie sind gegen normal sensible und resistente Arten sowie gegen alle oder einezlne Entwicklungsstadien wirksam. Zu den oben erwähnten Schädlingen gehören:

Aus der Ordnung der Isopoda z.B. Oniscus asellus, Armadillidium vulgare, Porcellio scaber.
Aus der Ordnung der Diplopoda z.B. Blaniulus guttulatus.
Aus der Ordnung der Chilopoda z.B. Geophilus carpophaus, Scutigera spec.
Aus der Ordnung der Symphyla z.B. Scutigerella immaculata.
Aus der Ordnung der Thysanura z.B. Lepisma saccharina.
Aus der Ordnung der Collembola z.B. Onychiurus armatus.
Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.
Aus der Ordnung der Dermaptera z.B. Forficula auricularia.
Aus der Ordnung der Isoptera z.B. Reticulitermes spp..
Aus der Ordnung der Anoplura z.B. Phylloxera vastatrix, Pemphigus spp., Pediculus humanus corporis, Haematopinus spp., Linognathus spp.
Aus der Ordnung der Mallophaga z.B. Trichodectes spp., Damalinea spp.
Aus der Ordnung der Thysanoptera z.B. Hercinothrips fermoralis, Thrips tabaci.

Aus der Ordnung der Heteroptera z.B. Eurygaster spp., Dysdercus intermedius, Piesma quadrata, Cimex lectularius, Rhodnius prolixus, Triatoma spp.
Aus der Ordnung der Homoptera z.B. Aleurodes brassicae, Bemisia tabaci, Trialeurodes vaporariorum, Aphis gossypii, Brevicoryne brassicae, Cryptomyzus ribis, Doralis fabae, Doralis pomi, Eriosoma lanigerum, Hyalopterus arundinis, Macrosiphum avenae, Myzus spp.,Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp.
Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp. Earias insulana, Heliothis spp., Laphygma exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Cacoecia podana, Capua reticulana, Choristoneura fumiferana, Clysia ambiguella, Homona magnanima, Tortrix viridana.
Aus der Ordnung der Coleoptera z.B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp.,

Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.

Aus der Ordnung der Hymenoptera z.B. Diprion spp., Hoplocampa spp., Lasius spp., Monomorium pharaonis, Vespa spp.

Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z.B. Xenopsylla cheopis, Ceratophyllus spp..

Aus der Ordnung der Arachnida z.B. Scorpio maurus, Latrodectus mactans.

Aus der Ordnung der Acarina z.B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp..

Zu den pflanzenparasitären Nematoden gehören Pratylenchus spp., Radopholus similis, Ditylenchus dipsaci, Tylenchulus semipenetrans, Heterodera spp., Meloidogyne spp., Aphelenchoides spp., Longidorus spp., Xiphinema spp., Trichodorus spp..

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chloräthylene oder Methylenchlorid, aliphatische Kohlenwasserstoffe, wie Cyclohexan oder Paraffine, z.B. Erdölfraktionen, Alkohole, wie Butanol oder Glycol sowie deren Äther und Ester, Ketone, wie Aceton, Methyläthylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgas, wie Halogenkohlenwasserstoffe sowie Butan, Propan, Stickstoff

und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z.B. nichtionogene und anionische Emulgatoren, wie Polyoxyäthylen-Fettsäure-Ester, Polyoxyäthylen-Fettalkohol-Äther, z.B. Alkylarylpolyglykol-äther, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azol-Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die Anwendung der erfindungsgemäßen Wirkstoffe erfolgt in Form ihrer handelsüblichen Formulierungen und/oder den aus diesen Formulierungen bereiteten Anwendungsformen.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 100 Gew.-% Wirkstoff, vorzugsweise zwischen 0,01 und 10 Gew.-% liegen.

Die Anwendung geschieht in einer den Anwendungsformen angepaßten üblichen Weise.

Bei der Anwendung gegen Hygiene- und Vorratsschädlinge zeichnen sich die Wirkstoffe durch eine hervorragende Residualwirkung auf Holz und Ton sowie durch eine gute Alkalistabilität auf gekälkten Unterlagen aus.

Beispiel A

Grenzkonzentrations-Test / Wurzelsystemische Wirkung

Testinsekt: Phaedon cochleariae-Larven
Lösungsmittel: 3 Gewichtsteile Aceton
Emulgator: 1 Gewichtsteil Alkylarylpolyglykoläther

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit Boden vermischt. Dabei spielt die Konzentration des Wirkstoffs in der Zubereitung praktisch keine Rolle, entscheidend ist allein die Wirkstoffgewichtsmenge pro Volumeneinheit Boden, welche in ppm (=mg/l) angegeben wird. Man füllt den behandelten Boden in Töpfe und bepflanzt diese mit Kohl (Brassica oleracea). Der Wirkstoff kann so von den Pflanzenwurzeln aus dem Boden aufgenommen und in die Blätter transportiert werden.

Für den Nachweis des wurzelsystemischen Effektes werden nach 7 Tagen ausschließlich die Blätter mit den obengenannten Testtieren besetzt. Nach weiteren 2 Tagen erfolgt die Auswertung durch Zählen oder Schätzen der toten Tiere. Aus den Abtötungszahlen wird die wurzelsystemische Wirkung des Wirkstoffs abgeleitet. Sie ist 100 %, wenn alle Testtiere abgetötet sind und 0 %, wenn noch genau so viele Testinsekten leben wie bei der unbehandelten Kontrolle.

Bei diesem Test zeigen z.B. die folgenden Verbindungen der Herstellungsbeispiele überlegene Wirkung gegenüber dem Stand der Technik: 1.

Beispiel B

Phaedon-Larven-Test

Lösungsmittel: 3 Gewichtsteile Dimethylformamid
Emulgator: 1 Gewichtsteil Alkylarylpolyglykoläther

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Meerrettichblattkäfer-Larven (Phaedon cochleariae) besetzt, solange die Blätter noch feucht sind.

Nach der gewünschten Zeit wird die Abtötung in % bestimmt. Dabei bedeutet 100 %, daß alle Käfer-Larven abgetötet wurden; 0 % bedeutet, daß keine Käfer-Larven abgetötet wurden.

Bei diesem Test zeigen z.B. die folgenden Verbindungen der Herstellungsbeispiele überlegene Wirksamkeit gegenüber dem Stand der Technik: 1.

Beispiel C

Myzus-Test

Lösungsmittel: 3 Gewichtsteile Dimethylformamid
Emulgator: 1 Gewichtsteil Alkylarylpolyglykoläther

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea), die stark von der Pfirsichblattlaus (Myzus persicae) befallen sind, werden durch Tauchen in die Wirkstoffzubereitung der gewünschten Konzentration behandelt.

Nach der gewünschten Zeit wird die Abtötung in % bestimmt. Dabei bedeutet 100 %, daß alle Blattläuse abgetötet wurden; 0 % bedeutet, daß keine Blattläuse abgetötet wurden.

Bei diesem Test zeigen z.B. die folgenden Verbindungen der Herstellungsbeispiele überlegene Wirksamkeit gegenüber dem Stand der Technik: 1.

Herstellungsbeispiele 

Beispiel 1

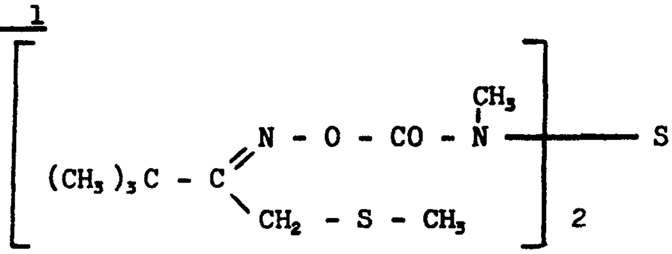

60g (0,37 Mol) 3,3-Dimethyl-1-methylthio-2-oximino-butan und 34,2g (0,18 Mol) N,N'-Bis-(fluorcarbonyl)-thio-bis-methylamin werden in 250 ml Dioxan gelöst und unter Rühren tropfenweise mit 37,3g (0,37 Mol) Triethylamin versetzt. Man läßt 48 Stunden bei Raumtemperatur rühren und gießt dann das Reaktionsgemisch in 800 ml Wasser. Die organische Phase wird mit Methylenchlorid extrahiert, über Natriumsulfat getrocknet und eingeengt. Der Rückstand kristallisiert nach Verrühren mit Petrolether. Man erhält 42g (52,5 % der Theorie) N,N'-Bis-(3,3-dimethyl-1-methylthio-2-oximinocarbonyl-butan)-thio-bis-methyl-amin vom Schmelzpunkt 87-93°C.

In analoger Weise können die Verbindungen der nachfolgenden Tabelle 1 erhalten werden.

Tabelle 1

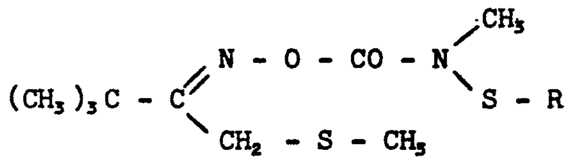

| R | Schmelzpunkt(°C) |
|---|---|
| 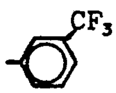 | |
| $-CCl_3$ | |
| 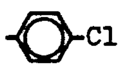 | Oel |
| 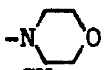 | |
| 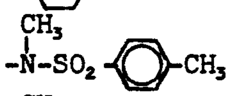 | |
| 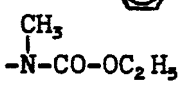 | |
| 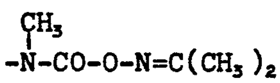 | |
| 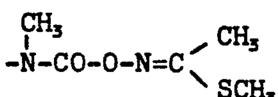 | |
| 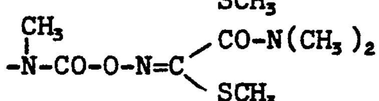 | |
| 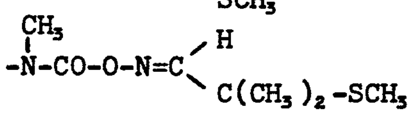 | |

Patentansprüche

1. N-sulfenylierte Carbamoyloximino-1-methylthio-butane der allgemeinen Formel (I)

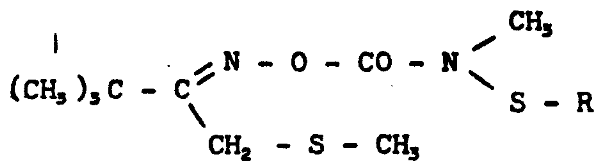 (I)

in welcher

R für Alkyl, Halogenalkyl, gegebenenfalls substituiertes Phenyl oder die Gruppe $-NR^1R^2$ sowie für den gleichen Rest steht, an den die Gruppe -SR gebunden ist,

$R^1$ für Alkyl steht,

$R^2$ für Alkyl, Halogencarbonyl, gegebenenfalls substituiertes Phenylsulfonyl oder die Gruppierungen $-CO-O-R^3$ und $-CO-NR^4R^5$ steht sowie

$R^1$ und $R^2$ gemeinsam mit dem N-Atom für einen Ring stehen, der gegebenenfalls ein weiteres Heteroatom enthält,

$R^3$ für Alkyl, Alkenyl, Alkinyl, gegebenenfalls substituiertes Aryl oder die Gruppierung $-N=CR^6R^7$ steht,

$R^4$ und $R^5$ gleich oder verschieden sind und für Alkyl stehen oder gemeinsam mit dem N-Atom für einen Ring stehen, der gegebenenfalls ein weiteres Heteroatom enthält,

$R^6$ und $R^7$ gleich oder verschieden sind und für Wasserstoff, Alkyl, Alkoxy, Alkylthio, Alkoxyalkyl, Alkylthioalkyl, Dialkylaminocarbonyl und Alkoxycarbonyl oder gemeinsam mit dem C-Atom für einen Dithian-Ring stehen.

2. Verfahren zur Herstellung der N-sulfenylierten Carbamoyloximino-1-methylthio-butane der Formel (I), dadurch gekennzeichnet, daß man

a) das Oxim der Formel (II)

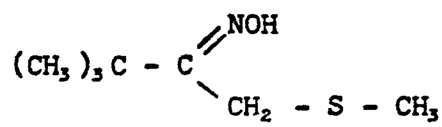

(II)

mit sulfenylierten Carbamoylhalogeniden der Formel (III)

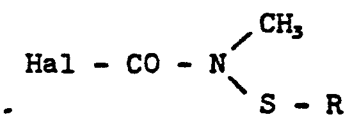

(III)

in welcher

R die oben angegebene Bedeutung hat und

Hal für Fluor oder Chlor steht,

in Gegenwart eines Verdünnungsmittels und eines Säurebindemittels umsetzt, oder

b) gegebenenfalls die nach dem Verfahren (a) erhältlichen sulfenylierten Oximcarbamate der Formel (IV)

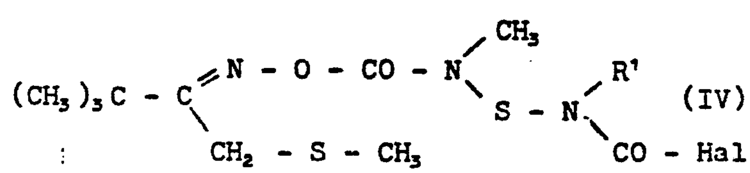

(IV)

in welcher

$R^1$ und Hal die oben angegebene Bedeutung haben,

mit Verbindungen der Formel (V)

$$H\text{-}R^8 \qquad (V)$$

in welcher

$R^8$ für die Gruppierungen $-OR^3$ und $-NR^4R^5$ steht und $R^3$, $R^4$ und $R^5$ die oben angegebene Bedeutung haben,

in Gegenwart eines Verdünnungsmittels und eines Säurebindemittels umsetzt.

3. Insektizide Mittel, gekennzeichnet durch einen Gehalt an mindestens einem N-sulfenylierten Carbamoyloximino-1-methylthio-butan der Formel (I) gemäß Anspruch 1.

4. Verwendung von N-sulfenylierten Carbamoyloximino-1-methylthio-butanen der Formel (I) gemäß Anspruch 1 zur Bekämpfung von Insekten.

5. Verfahren zur Bekämpfung von Insekten, dadurch gekennzeichnet, daß man N-sulfenylierte Carbamoyloximino-1-methylthio-butane der Formel (I) gemäß Anspruch 1 auf Insekten und/oder ihren Lebensraum einwirken läßt.

6. Verfahren zur Herstellung insektizider Mittel, dadurch gekennzeichnet, daß man N-sulfenylierte Carbamoyloximino-1-methylthio-butane der Formel (I) gemäß Anspruch 1 mit Streckmitteln und/oder oberflächenaktiven Mitteln vermischt.

0007987

Nummer der Anmeldung

Europäisches Patentamt

# EUROPÄISCHER RECHERCHENBERICHT

EP 79 101 944.1

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| D,X | DE - A1 - 2 409 463 (BAYER)<br>* Ansprüche 1, 2, 3 *<br>-- | 1-3 |
| D | DE - A - 2 216 838 (DIAMOND SHAMROCK)<br>* Ansprüche 1 und 3 *<br>-- | 1,3 |
| D | DE - A1 - 2 654 282 (UNION CARBIDE)<br>* Anspruch 1; Seite 10, Absatz 3 *<br>-- | 1,3 |
| A | DE - A1 - 2 530 439 (CIBA-GEIGY)<br>-- | |
| A | DE - A 2 106 300 (BAYER)<br>-- | |
| A | DE - A1 - 2 727 614 (CIBA-GEIGY)<br>---- | |

KLASSIFIKATION DER ANMELDUNG (Int.Cl.3)

C 07 C 149/14
A 01 N 47/24
C 07 D 295/22

RECHERCHIERTE SACHGEBIETE (Int. Cl.3)

A 01 N 47/24
C 07 C 149/14
C 07 C 161/00
C 07 D 295/22

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| Berlin | 10-10-1979 | STOOS |